**Europäisches Patentamt**

**European Patent Office**

**Office européen des brevets**

(19)

(11) Publication number : **0 530 006 A1**

(12)

# EUROPEAN PATENT APPLICATION

(21) Application number : **92307763.0**

(22) Date of filing : **26.08.92**

(51) Int. Cl.$^5$ : **A61N 1/372, H01L 27/22**

(30) Priority : **26.08.91 US 750143**

(43) Date of publication of application :
**03.03.93 Bulletin 93/09**

(84) Designated Contracting States :
**DE FR GB IT NL SE**

(71) Applicant : **MEDTRONIC, INC.**
**7000 Central Avenue N.E.**
**Minneapolis, Minnesota 55432-3576 (US)**

(72) Inventor : **Wahlstrand, John**
**685 Lake Pine Drive**
**Shoreview, Minnesota 55126 (US)**
Inventor : **Thompson, David**
**1660 Onondaga Street N.E.**
**Fridley, Minnesota 55432 (US)**
Inventor : **Nelson, Gary**
**1322 Colwyn Drive**
**Schaumburg, Illinois 60194 (US)**

(74) Representative : **Tomlinson, Kerry John et al**
**Frank B. Dehn & Co. European Patent**
**Attorneys Imperial House 15-19 Kingsway**
**London WC2B 6UZ (GB)**

(54) **Magnetic field sensor for implantable medical device.**

(57)   An implantable medical device including a circuit responsive to magnetic field comprising a first P-channel split-drain FET (42) having first and second split-drain parts (50, 54) and a second N-channel split-drain FET (44) having third and fourth split-drain parts (56, 52), said first and fourth split-drain parts being connected together such that an applied magnetic field causes an increase in the current in said first drain (50) and a decrease in the current in said fourth drain (52), accompanied by a corresponding decrease and increase in the current in said second and third drain. In one embodiment, said second and third drain are connected together and the voltage difference between the connected drain parts (58,60) is monitored.

Fig. 3

EP 0 530 006 A1

This invention relates generally to the field of implantable medical devices, and more particularly to a sensor for detecting the application of a magnetic field to a medical device

In the field of medical devices which are implanted within the body of a human patient, such as implantable cardiac stimulators and the like, it is often desirable that certain operational parameters of the device be altered in a non-invasive (i.e. non-surgical) means. Various means of non-invasive communication with implanted devices have been previously disclosed. Since the introduction of demand cardiac pacemakers, one of the most common methods of non-invasive alteration of operational parameters employs a magnetically actuated reed switch contained within the implanted devices. The magnetic reed switch consists of a hermetically sealed cylindrical encapsulation housing two metallic reeds. The metallic reeds are disposed within the encapsulation such that when a sufficiently strong magnetic force is applied to the implanted device from outside the patient's body, the magnetic force draws the two reeds into electrical contact with one another, thereby completing an electrical circuit. When the magnetic field is withdrawn, the reeds separate, breaking the electrical circuit. Such an arrangement is disclosed, for example, in U.S. Patent No. 3, 805, 796 entitled "Implantable Cardiac Pacer Having Adjustable Parameters" issued to Terry, Jr. et al. on April 23, 1974, U.S. Patent No. 3,920,005, entitled "Evaluation System for Cardiac Stimulators" issued to Gombrich et al. on November 18, 1975, and U.S. Patent No. 4,066,086 entitled "Programmable Body Stimulator" issued to Alferness et al. on January 3, 1978, each assigned to the assignee of the present invention.

Reed switch closure is used to enable asynchronous operation for follow-up and trans-telephonic evaluation of the implanted pacemaker. In addition, rate and mode changes which occur upon reed switch closure are used to indicate device function and battery status. More recently, external devices have been developed which communicate with implanted devices via radio-frequency (RF) telemetry. An RF telemetry link allows two-way communication between an external device and an implanted device, and enables a wider range of operational parameters to be externally programmed. However, the use of an RF link does not necessarily eliminate the need for a magnetically-actuated reed switch. In U.S. Patent No. 4,250, 833 entitled "Digital Cardiac Pacemaker With Refractory, Reversion and Sense Reset Means" issued to David L. Thompson on February 17, 1981, for example, the disclosed pacemaker cannot receive and process external RF telemetry signals until the reed switch is closed. Such an arrangement ensures that the implanted device will not be unintentionally re-programmed by extraneous RF signals to which the patient may be exposed.

Although magnetic reed switches are commonly employed in implanted devices, there are nonetheless several known problems associated with them. Reed switches are are typically the only mechanical devices with moving pairs in a pacemaker, making them more susceptible than the pacemaker's electronic components to damage or mechanical failure such as might result from vibration or mechanical shock. Although the glass encapsulation affords some measure of protection to the reeds, the capsule itself is susceptible to breakage. Furthermore, advances in electronic technology have resulted in progressively smaller pacemakers, so that the reed switch itself must be made very small, thereby adding to its fragility. Also, thin pacemakers, typically on the order of six to eight millimeters thick, prevent reed switches from being oriented in a preferred orthogonal orientation to the large flat surface area of the pacemaker case. Failure of the reed switch is particularly undesirable in the context of implantable devices, since replacement of such devices involves a surgical procedure.

While a reed switch must be sensitive enough to be responsive to an externally applied magnetic field, it is important that the switch not be so sensitive that it is responsive to every magnetic field to which the patient may be exposed in daily activity. As a result, the manufacturing tolerances for reed switches are low, making manufacturing costs high.

One attempt to overcome the disadvantages of a reed switch has been described in U.S. Patent No. 3,766,928 issued to Goldberg et al. This patent discloses a potentiometer which is affixed to a small diametrically magnetized disc magnet. A second magnet is rotated outside the patient to cause the disc magnet to rotate and turn the potentiometer. This method itself has numerous disadvantabes including the fact that it is also mechanical, and very small, and thus prone to breakabe. In addition, the manipulation of the second magnet in order to adjust the potentiometer is more difficult and complex than the manipulation of the magnet required to actuate a reed switch.

Another attempt to overcome the disadvantages of a reed switch has been described in U. S. Patent No. 4, 301, 804 entitled " Pacemaker With Hall Effect Externally Controlled Switch" issued on November 24, 1981 to Thompson et al. and assigned to the assignee of the present invention. This patent discloses a pacemaker in which a circuit produces a strobe signal which is used to turn on a current flow through a Hall effect element once each pacemaker pulse cycle for a selected period of time. The presence of an external magnetic field alters the electrical properties of the Hall effect element (typically implemented in a bipolar integrated circuit fabrication process), so that a positive voltage is provided to the pacemaker circuitry when the element is strobed. While the Hall effect element is not a mechanical device, and is in that respect preferable to a reed switch, the Hall effect element has proven to be less sensitive than a reed switch, requires expensive processing and packaging, and is not compatible with standard linear CMOS processing which is preferentially used in implantable medical devices.

As an alternative to using mechanical reed-switches or Hall effect elements to detect and measure magnetic fields, it has been proposed in the prior art to employ split-drain field-effect transistors, sometimes called MAGFETs, for this purpose. Although similar to a conventional field-effect transistor (FET), the drain of a MAGFET is split into two isolated halves. Application of a magnetic field to a MAGFET device gives rise to a differential in the currents in the two split-drain-halves, the extent of this differential being directly proportional to the strength of the applied magnetic field. Although MAGFETs, like Hall-effect devices, have the advantage of being solid-state devices, some problems with prior MAGFETs are known. One problem relates to the sensitivity of these devices to magnetic fields. Typically, the gain constant of a MAGFET is small, i.e. the current differential between the drain current in respective drain-halves is rather slight for a given change in magnetic field intensity. In order for a MAGFET to be readily utilized to detect and measure an external magnetic field, it is desireable to maximize the gain constant of the MAGFET. It is has been taught in the prior art that the gain constant of a MAGFET can be increased by raising the bias current of the MAGFET in steady-state . (See, e.g., Misra et al., "A Novel High Gain MOS Magnetic Field Sensor", *Sensors and Actuators*, pp. 213 - 221 ( 1986)). In addition, many prior art references discuss operation of MAGFETs with five- to ten-volt power supplies and 10- to 620-micro-amp bias currents. (See, e. g., Misra, "A Novel CMOS Magnetic Field Sensor Array", *IEEE Journal of Solid-State Circuits*, v. 25, no. 2, April 1990, pp. 623 - 625; Nathan et al., "Design of a CMOS Oscillator with Magnetic-Field Frequency Modulation", *IEEE Journal of Solid-State Circuits*, v. SC-22, n. 2, April 1987, pp. 230 - 232.) Such power supply voltages and bias currents are not available in implantable pacemakers, however, which must operate with one- to three-volt power supplies and ten to one-hundred nano-amp bias currents.

Another method of the prior art for achieving increased sensitivity to magnetic fields involves using an array of MAGFET devices in a tree arrangement, and combining the drain-halves from the individual MAGFETs such that the effect of the magnetic field is measured by the additive effect of the current differential in each of the individual split-drain pairs. (See, e.g., Misra et al., "A Novel High Gain MOS Magnetic Field Sensor", *Sensors and Actuators*, pp. 213 - 221 (1986).) This solution, however, requires a higher-level supply voltage, since the threshold voltages of MAGFETs coupled in this manner are additive. Moreover, the use of multiple MAGFET devices results in a corresponding increase in total circuit area, and an increase in current drain for the sensor. Thus, prior art techniques for increasing the gain constant of MAGFET devices have proven unsuitable for application in implantable medical devices, which must maintain small size, long-term stability and reliability, minimal power-supply voltage levels and minimal current drain.

It is accordingly a feature of the present invention that a non-mechanical device is provided which is sufficiently sensitive to external magnetic fields to be useful in the context of an implantable medical device.

It is another feature of the present invention that the magnetic sensor is operable at the low supply voltages and bias currents available in an implanted medical device.

It is still another feature of the present invention that the magnetically-actuated device is simple to produce using conventional CMOS processing techniques.

It is yet another feature of the present invention that the magnetically actuated device remains sufficiently sensitive to external magnetic fields even when the device is provided with the low supply voltages and bias currents of an implantable medical device.

The foregoing and other features of the present invention are realized in a CMOS device comprising a cross-coupled arrangement of two split-drain field-effect transistors (MAGFETs). In the absence of an external magnetic field, the total drain current in each MAGFET is evenly divided between each half of the split-drain in that MAGFET. An external magnetic field directed perpendicularly to the plane of the channel in each MAGFET gives rise to a Lorentz force exerted on injected carriers in the MAGFET, causing a variation in the drain current in each half of the split-drain. Since the total drain current in each MAGFET remains the mme, an increase in the drain current in one half of a MAGFET's split-drain will be accompanied by a corresponding decrease in the drain current in the other half of the split-drain. In this way, the presence and strength of an external magnetic field is manifested as a voltage or current differential between the two halves of the split-drain in each MAGFET. A high-gain differential amplifier situated between the cross-coupled MAGFETS can therefore produce an output indicative of the application of an external magnetic field.

In accordance with one embodiment of the invention, a novel cross-coupling of the split-drains results in a doubling of the voltage differential resulting from application of a given magnetic field. The enhanced voltage differential and low current drain of a device in accordance with one embodiment of the invention makes the device especially suitable for use implanted pacemakers and the like.

There now follows, by way of example only, a detailed description of embodiments of the present invention, in conjunction with the accompanying drawings, wherein:

Figs. 1a and 1b are perspective views of split-drain FETs utilized in a magnetic sensor made in accordance with one embodiment of the present invention;

Figs. 2a and 2b are alternative schematic representations of the split-drain FET of Fig. 1;

Fig. 3 is a schematic diagram of a illustrating the cross-coupling of split-drain FETs in accordance with the present invention;

Fig. 4 is a schematic diagram of a magnetic sensor circuit in accordance with one embodiment of the present invention;

Fig. 5 is a schematic diagram of the P-channel MAGFET from the circuit of Fig. 4;

Fig. 6 is a schematic diagram of the N-channel MAGFET from the circuit of Fig. 4;

Fig. 7 is a schematic diagram of an alternative embodiment of a magnetic sensor circuit in accordance with the present invention;

Fig. 8 is a schematic diagram of the multiplexer from the circuit of Fig. 7;

Fig. 9 is a schematic diagram of an alternative embodiment of a magnetic sensor in accordance with the present invention;

Fig. 10 is a schematic diagram of the P-channel MAGFET from the circuit of Fig. 9;

Fig. 11 is a schematic diagram of the N-channel MAGFET from the circuit of Fig. 9; and

Fig. 12 is a schematic diagram of an alternative embodiment of a magnetic sensor in accordance with the present invention.

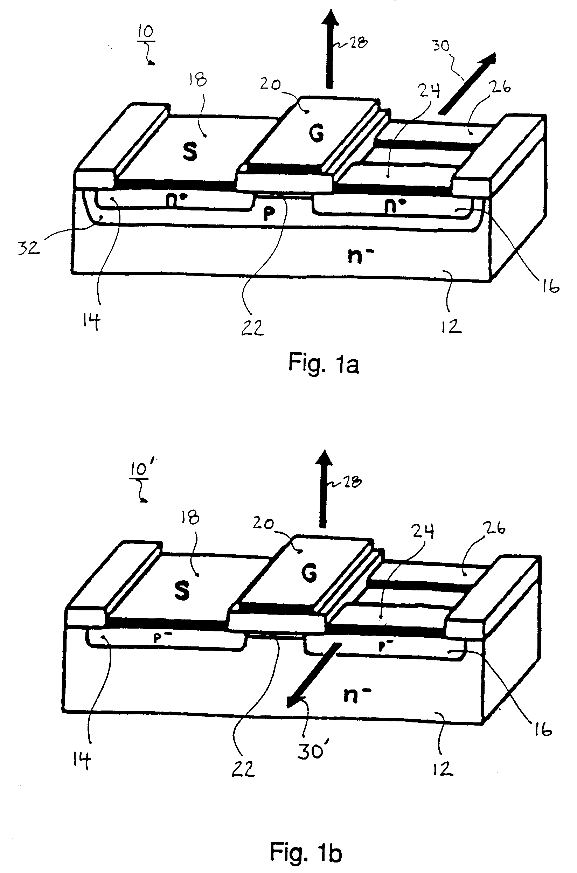

Referring to Fig. 1a, a three-dimensional view of an N-channel split-drain MAGFET 10 is shown. As in conventional N-channel FETs, split-drain MAGFET 10 comprises an N-type substrate 12 having a P-well 32 formed therein. P-well 32, in turn, has N-type source and drain regions 14 and 16, respectively, formed therein. The metal source 18 and gate 20 are also conventional FET structures. Gate 20 covers a channel region 22 disposed between source region 14 and drain region 16. Unlike a conventional FET, however, the metal drain of MAGFET 10 is split into two distinct halves, 24 and 26, which are electrically isolated from one another. Fig. 1b is a three-dimensional view of a similar P-channel split-drain MAGFET 10' in which components identical to those of Fig. 1a have retained identical reference numerals. MAGFET 10' differs from MAGFET 10 only in the wafer processing steps and the absence of a P-well.

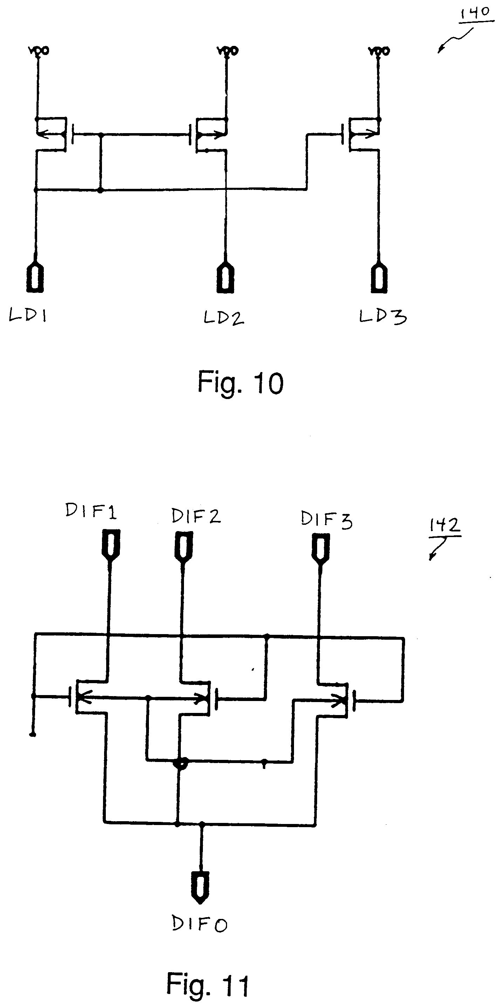

As would be appreciated by one of ordinary skill in the semiconductor art, if a magnetic field is applied to MAGFET 10 in the direction indicated by arrow 28, perpendicular to the plane of channel region 22, a Lorentz force is exerted on injected carriers. The direction of the Lorentz force can be determined using the so-called "right-hand rule": if the thumb of a person's right hand is pointed in the direction of the magnetic field, the Lorentz force resulting from that magnetic field will be exerted in the direction of the curled fingers of that person's right hand. In FET 10 of Fig. 1a, therefore, a magnetic field in the direction of arrow 28 will give rise to a Lorentz force directed in the direction indicated by arrow 30, perpendicular to arrow 28 and in the perpendicular direction between split-drain-half 24 and split-drain -half 26. In FET 10' of Fig. 1b, a magnetic field in the direction of arrow 28 will give rise to a Lorentz force directed in the direction indicated by arrow 30', perpendicular to arrow 28, in the perpendicular direction between split-drain half 24 and split-drain half 26, and in the opposite direction from arrow 30 in Fig. 1a. The opposing directions of arrows 30 and 30' of Figures 1a and 1b is due to electrons and holes being the predominant carriers in Figure 10 and 10', respectively.

As a result of this Lorentz force, the drain current of MAGFET 10, which is, as noted above, normally evenly divided between the split-drain-halves 24 and 26, will be concentrated more in the split drain half 26 and less in split drain half 24. In particular, if MAGFET 10 is biased such that a total drain current $I_T$-amps flows through drains-halves 24 and 26, in the absence of an external magnetic field a current of ($\frac{1}{2}I_T$-amps will flow in drain-half 24, and $\frac{1}{2}/I_T$-amps in drain-half 26. When an external magnetic field is applied, the total drain current IT-amps remains the same, but some amount ($\frac{1}{2}I_T$ + i)-amps will flow in drain-half 26, while some amount ($\frac{1}{2}I_T$ - i)-amps will flow in drain-half 24. This results in a difference of 2i-amps between the current in drain-halves 24 and 26; a measurement of the 2i-amp difference between the currents in the respective drain-halves 24 and 26 can thus be used to determine the presence and strength of an external magnetic field.

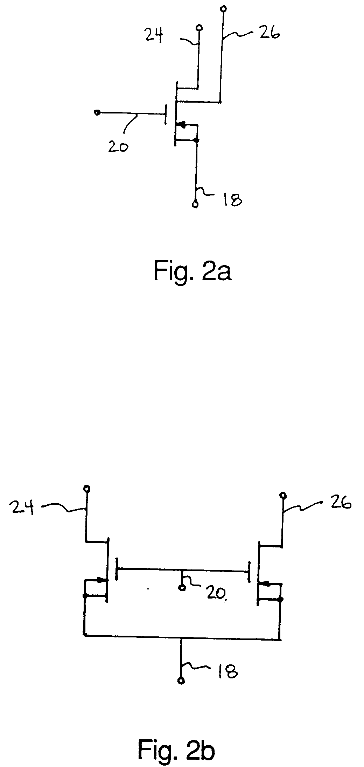

A circuit symbol used to schematically represent a MAGFET 10 is shown in Fig. 2a. An alternative schematic representation of MAGFET 10 is shown in Fig. 2b. In the representations of Figs. 2a and 2b, as in Fig. 1, reference numeral 18 denotes the source terminal of MAGFET 10, reference numeral 20 denotes the gate terminal, and reference numerals 24 and 26 denote the two split-drain halves.

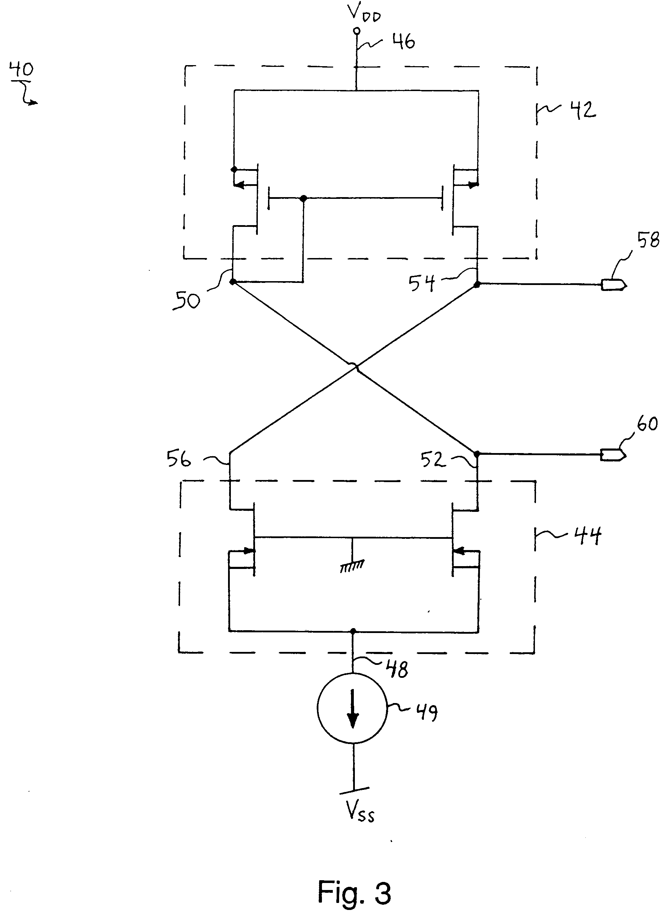

Turning now to Figure 3, a magnetic sensor 40 in accordance with one embodiment of the present invention is shown in schematic form, wherein two MAGFETS 42 and 44 are represented using the convention of Fig. 2b. Sensor 40 consists of a P-channel MAGFET indicated by dashed line 42, and an N-channel MAGFET indicated by dashed line 44. Source terminal 46 of MAGFET 42 receives a power supply voltage $V_{DD}$, provided, for example, from a battery. Source terminal 48 of MAGFET 44 is coupled to a regulated current source 49.

The silicon substrate on which sensor 40 is constructed is coupled to a negative supply voltage $NV_{DD}$, which shall variously be referred to herein and in the Figures as $NV_{DD}$ or $V_{SS}$. Split-drain -half 50 of MAGFET 42 is coupled to split-drain-half 52 of MAGFET 44, while split-drain-half 54 of MAGFET 42 is coupled to split-drain-half 56 of MAGFET 44. A first output terminal 58 is coupled to the drain-halves 54 and 56, while a second output terminal 60 is coupled to the drain-halves 50 and 52.

MAGFET 42 functions in sensor 40 as an active load. In the absence of an external magnetic field, the currents in respective split-drain-halves 50 and 54 of MAGFET 42 are equal, so that the voltages on respective output terminals 58 and 60 are likewise equal, with a voltage level near the midpoint between $V_{DD}$ and $V_{SS}$. MAGFETs 42 and 44 are physically arranged relative to one another on the substrate such that an external magnetic field applied to sensor 40 will cause drain current in the respective MAGFETs 42 and 44 to be "crowded", by Lorentz force, either into split-drain-halves 50 and 56, or into split-drain-halves 52 and 54, depending upon the polity of the magnetic field.

In the case that an applied magnetic field causes current to be increased in split-drain-halves 50 and 56, a corresponding reduction in the currents in split-drain-halves 52 and 54 will also occur. Since the current in split-drain-half 50 is increased, while the current in split-drain-half 52 is decreased, the voltage at output terminal 60 will tend to increase; conversely, since the current in split-drain-half 54 is decreased, while the current in split-drain-half 56 in increased, the voltage at output terminal 58 will tend to decrease.

In the case that an applied magnetic field causes current to increase in split-drain-halves 52 and 54, a corresponding reduction in the currents in split-drain-halves 50 and 56 will occur. In this case, the voltage at output terminal 58 increases, while the voltage at output terminal 60 decreases. As would be appreciated by one of ordinary skill in the design of electronic circuits, a differential amplifier situated between the two output terminals 58 and 60 can be used to detect changes in the relative voltages of output terminals 58 and 60, thereby providing an indication of an externally applied magnetic field.

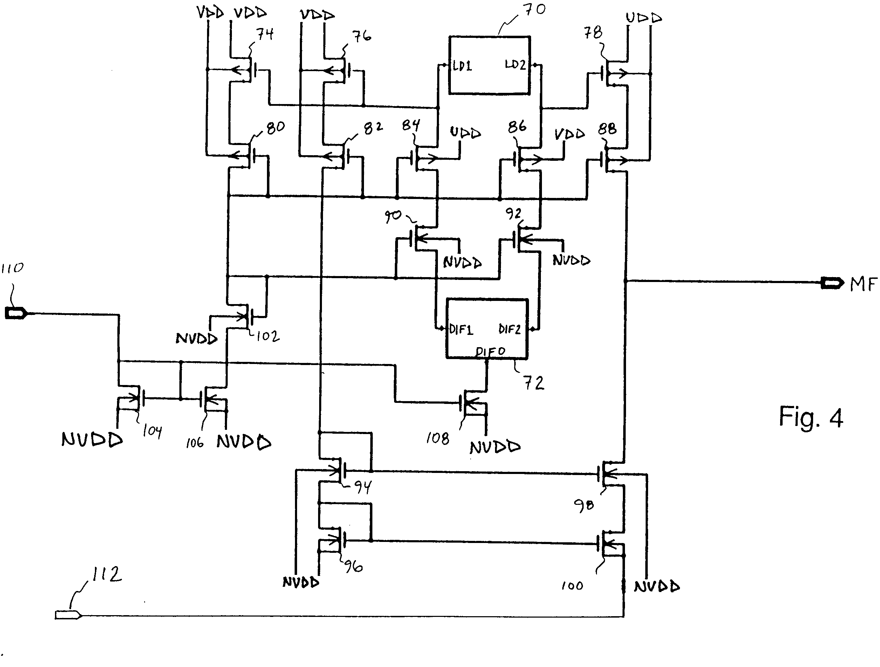

Turning now to Fig. 4, a schematic diagram of an implantable pacemaker circuit utilizing the magnetic sensor of the present invention is shown. The circuit of Fig. 4 comprises a very high gain differential amplifier coupled between the cross-coupled drains of two split-drain MAGFETs represented in Fig. 4 by blocks 70 and 72. Devices 74, 76, 78, 80, 82, 84, 86, 88, 90, 92, 94, 96, 98, 100, and 102 comprise a conventional differential amplifier, which produces an output voltage at the terminal designated MF. A current source is coupled to the circuit of Fig. 4 at terminal 110. The current source is applied to a current mirror comprising devices 104 and 106, and the regulated output current from the current mirror provides the proper current at the source terminal of MAGFET 72 via device 108. Trimming of wafer process variations is accomplished via a resistor (not shown) between the $NV_{DD}$ power supply and terminal 112. The resistor is adjusted while the device of Figure 4 is in a zero-intensity magnetic field.

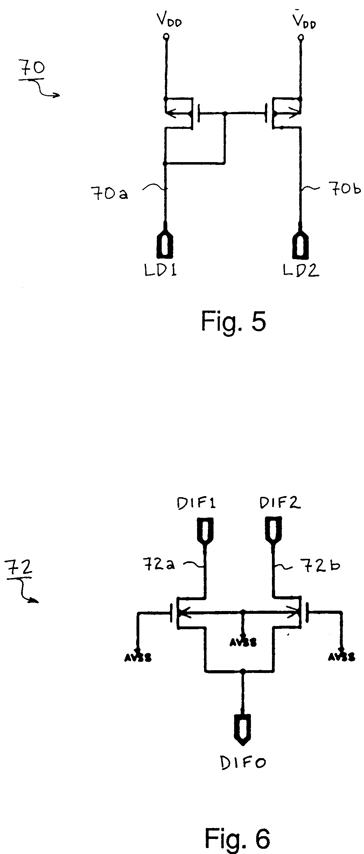

In Fig. 5, a schematic representation of MAGFET 70 is shown, using the representation of a split-drain transistor of Fig. 2b. In Fig. 5, one split-drain half of MAGFET 70 is designated as 70a, and the second split-drain half of MAGFET 70 is designated as 70b. MAGFET 70 is a P-channel device which serves as an active load device, in the same manner as MAGFET 42 served as the active load in the circuit of Fig. 3. MAGFET 70 is coupled to the circuit of Fig. 4 via terminals designated LD1 and LD2. In Fig. 6, a schematic representation of MAGFET 72 is shown, also using the representation of a split-drain transistor of Fig. 2b. In Fig. 6, one split-drain half of MAGFET 72 is designated as 72a, and the other split-drain half of MAGFET 72 is designated as 72b. MAGFET 72 is an N-channel device which is coupled to the circuit of Fig. 4 via terminals DIFO, DIF1, and DIF2.

As can be seen in Figs. 4, 5, and 6, split-drain half 70a of MAGFET 70 is coupled, via FETs 84 and 90, to split-drain half 72b of MAGFET 72. Similarly, split-drain half 70b of MAGFET 70 is coupled, via FETs 86 and 92, to split-drain half 72b of MAGFET 72. Subject, of course, to unavoidable CMOS process variation, the bias current through the split-drain halves 70a and 72a will be equal to the bias current through split-drain halves 70b and 72b. The differential amplifier comprising FETs 74 - 102 is biased such that in the absence of an external magnetic field, the voltage at the MF output terminal will be $V_{DD}$ (or $NV_{DD}$). Since the differential amplifier comprising FETs 74 - 102 has very high gain, the slight change in current through the split-drain halves resulting from the application of an external magnetic field causes the voltage at output terminal MF to change to $NV_{DD}$ (or $V_{DD}$).

As the circuit of Fig. 4 is part of the circuitry in an implantable pacemaker, it is essential that sensor 40 operates with low current drain and minimal positive and negative voltage supply levels $V_{DD}$ and $NV_{DD}$. At the same time, it is desireable to ensure that the circuit of Fig. 4 remain very sensitive to external magnetic fields. As previously noted, numerous references in the prior art have taught that the sensitivity of MAGFET devices to magnetic fields may be enhanced by increasing the power supply levels and bias current of the devices. However, the inventors have achieved sensitivities of 1200-volts/Tesla with the circuit of Fig. 4 operated with supply voltages of $\pm$ 1.8 to $\pm$ 3.0-volts and bias currents of ten to one-hundred nano-amps. This sensitivity is roughly one-thousand times previously published values. (See, e. g., Misra, et al. (1986)).

The sensitivity of the circuit to external magnetic fields, the gain constant of the differential amplifier circuitry, and the current drain characteristics of the circuit of Fig. 4 are also affected by the sizes of the various devices. A number of different combinations of device sizes are contemplated by the inventors. As would be appreciated by one of ordinary skill in the design of semiconductor circuits, the critical parameter of FET devices is the channel size, which may be designated as a ratio of channel width to channel length (W/L). In Table 1, a listing of the device sizes of one implementation of the circuit of Fig. 4 is provided.

## TABLE 1

| DEVICE | CHANNEL SIZE (width / length, in microns) |
| --- | --- |
| 70a/b | 158 / 316 |
| 72a/b | 158 / 316 |
| 74 | 38 / 4 |
| 76 | 38 / 4 |
| 78 | 38 / 4 |
| 80 | 38 / 4 |
| 82 | 38 / 4 |
| 84 | 38 / 4 |
| 86 | 38 / 4 |
| 88 | 38 / 4 |
| 90 | 15 / 4 |
| 92 | 15 / 4 |
| 94 | 15 / 4 |
| 96 | 15 / 4 |
| 98 | 60 / 12 |
| 100 | 60 / 12 |
| 102 | 15 / 4 |
| 104 | 2 x 60 / 12 |
| 106 | 60 / 12 |
| 108 | 2 x 60 / 12 |

Although the implementation of the circuit of Fig. 4 using channel sizes from Table 1 is presently preferred by the inventors, two additional implementations are contemplated. The first of these alternative embodiments employs devices according to Table 1, except that MAGFET devices 70a and 70b have width-to-length (w/l) ratios of 158μ/5μ instead of 158μ/316μ. In the second alternative embodiment, MAGFET devices 70a and 70b have w/l ratios of 3μ/316μ.

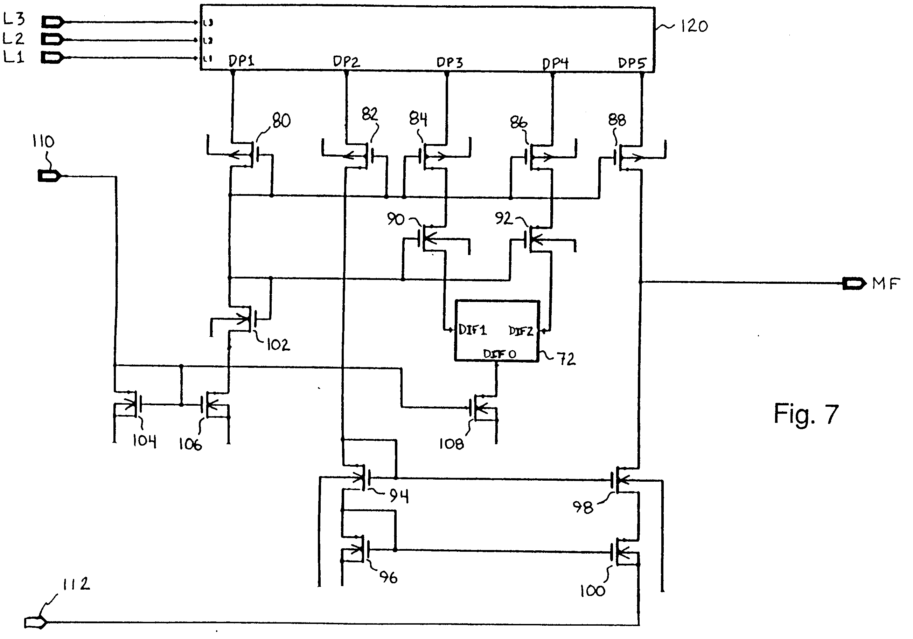

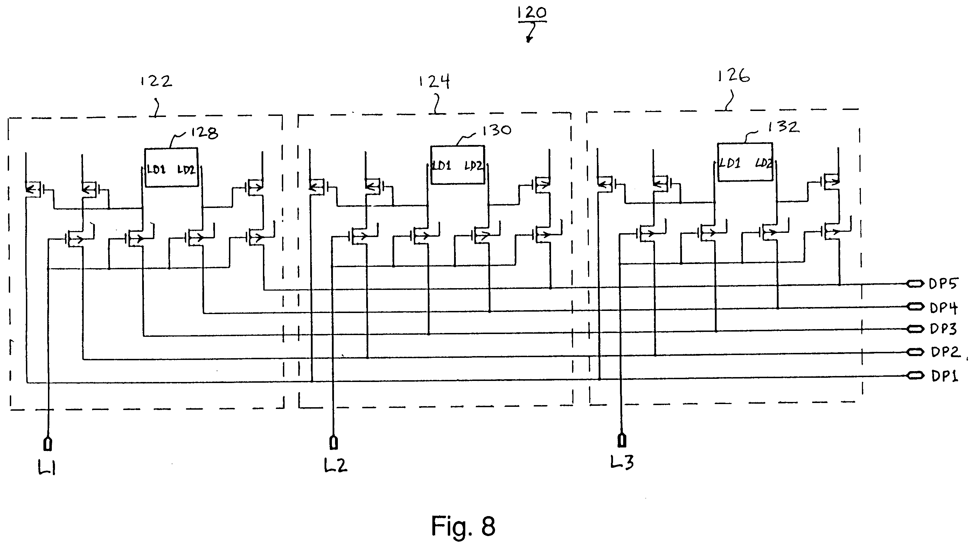

While the implementation of the circuit of Fig. 4 using the device sizes of Table 1 is deemed by the inventors to be a preferred method of practicing the present invention, it may nonetheless be desireable, in a given application, to consider alternative implementations. To this end, the inventors have employed a multiplexing arrangement, such as is shown in Fig. 7, which allows for several alternative combinations of active loads to be introduced into a magnetic sensor circuit in accordance with the present invention. In Fig. 7, those devices which are identical to corresponding devices in the implementation of Fig. 4 have retained identical reference numerals. The implementation of Fig. 7, however, further includes a multiplexer 120, which is shown in greater detail in Fig. 8. Multiplexer 120 receives input signals L1, L2, and L3, which are used to select one of several active loads to be introduced into the magnetic sensor circuit.

With reference to Fig. 8, each of the devices in multiplexer 120 has a w/l ratio of 38μ/4μ. Multiplexer circuitry 120 includes three separate active load portions, indicated in Fig. 8 by dashed lines 122, 124, and 126. Active load portion 122 includes a MAGFET device 128. Active load portion 124 includes a MAGFET device 130. Active load portion 126 includes a MAGFET device 132. As would be readily apparent to one of ordinary skill in the design of semiconductor circuits, applying a positive voltage on either line L1, L2, or L3 causes active load portion 122, 124, or 126, respectively, to be applied to the circuitry of Fig. 7 via terminals DP1, DP2, DP3, DP4, and DP5. Thus, by selecting either L1, L2, or L3, MAGFET 128, 130, or 132, respectively, is introduced into the magnetic sensor circuit of Fig. 7. In this way, various active load devices can be compared and tested for their suitability in a given application of the present invention, or, alternatively, a programmable sensitivity may be employed to allow a variable level of magnetic field intensity control.

The ability to program the sensitivity of the MAGFET will allow the use of varying levels of access to programming and diagnostic capabilities of implantable medical devices where the magnetic switch function is an interlock to initiate programming and telemetry. For example, company representatives aiding in the troubleshooting of a problem implant could have access to additional programmable parameters or device self-check routines, functions to which clinicians would be prevented access by means of a higher magnetic threshold. Additionally, patients with implanted neuro-stimulators are typically sent home with a patient programmer which enables the device's standard programming link but, via software, limits programmability. Undesirable programming could occur with the standard link being enabled. A programmable higher magnetic threshold can be used to allow very limited access to programmable parameters by a patient.

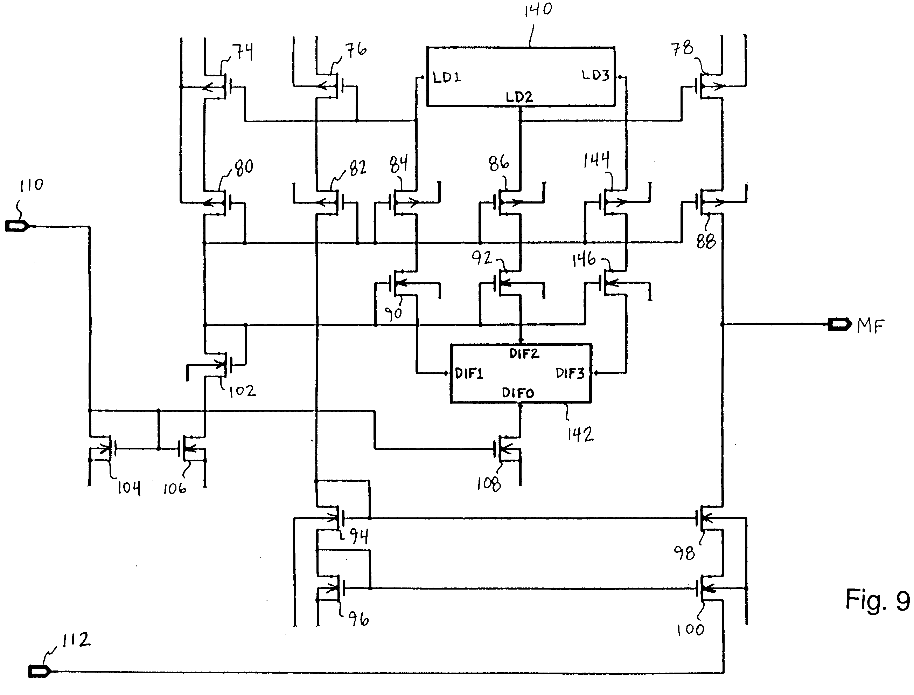

Still another embodiment of the present invention is shown in Figs. 9, 10 and 11, wherein devices which are identical to corresponding devices in the embodiment of Fig. 4 have retained identical reference numerals. The embodiment of Fig. 9 employs a tri-drain MAGFET devise 142 in place of the split-drain MAGFET 72 from Fig. 4. Active load device 140 and tri-drain MAGFET 142 are shown in greater detail in Figs. 10 and 11, respectively. Active load device 140 is a 316μ/316μ tri-drain P-channel MAGFET 150. MAGFET 142 is a 316μ/316μ tri-drain N-channel MAGFET. The differential amplifier circuitry in the embodiment of Fig. 9 includes additional FET devices 144 and 146, having channel sizes of 38μ/4μ and 15μ/4μ situated between terminal LD3 of active load 140 and terminal DIF3 of tri-drain MAGFET 142.

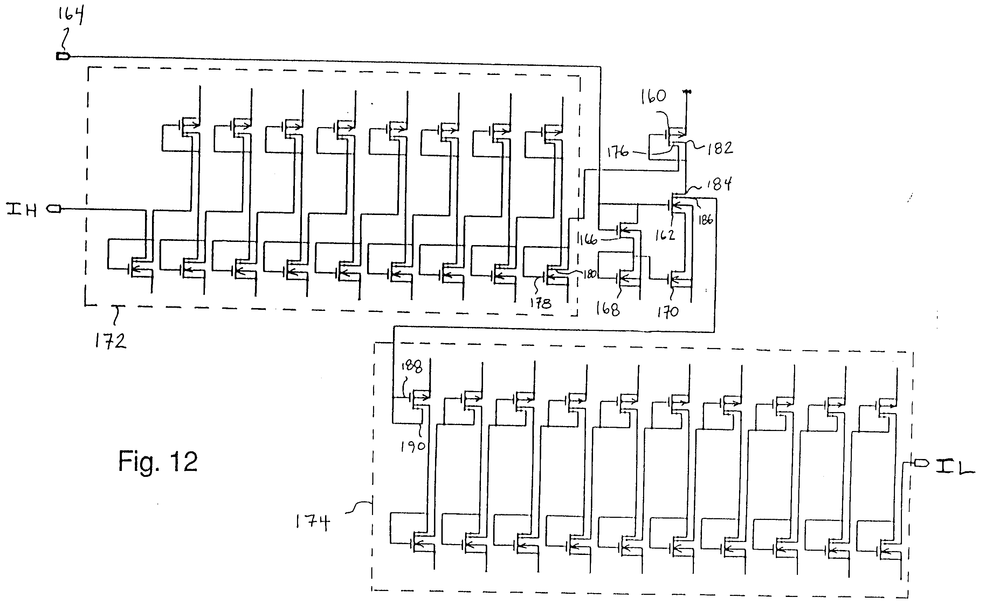

A final embodiment of the present invention is shown in Fig. 12. In the embodiment of Fig. 12, a plurality of split-drain MAGFETs are cascaded such that the current-crowding effects of an external magnetic field are enhanced. In Fig. 12, a P-channel MAGFET 160 has one of its split-drain halves coupled to the split-drain half of an N-channel MAGFET 162. A 10 nano-amp current source is coupled to the circuit at terminal 164, this current source being regulated by conventional FET devices 166, 168, and 170. The MAGFETs in Fig. 12 are coupled such that current passing through MAGFET 160 is conducted serially through each of the "upper" MAGFETs designated collectively in Fig. 12 within dashed line 172, while current through MAGFET 162 is conducted serially through each of the "lower" MAGFETs designated collectively in Fig. 12 within dashed line 174. As shown in Fig. 12, split-drain half 176 of MAGFET 160 is coupled to the gate 178 of the first of the "upper" MAGFETs 172, as well as to one split-drain half 180 of that MAGFET. The second split-drain half 182 of MAGFET 160 is coupled to split-drain half 184 of MAGFET 162. The second split-drain half 186 of MAGFET 162 is coupled to the gate 188 and one split-drain half 190 of the first of the "lower" MAGFETs 174.

As would be apparent to one of ordinary skill in semiconductor design, when an external magnetic field is applied to the circuit of Fig. 12, the current-crowding effect described above in conjunction with Fig. 1 results in an increase in the current through split-drain half 176 of MAGFET 160, and a corresponding decrease in the current through split-drain half 186 of MAGFET 162. The intercoupling of the "upper" MAGFETs 172 causes the increased current in split-drain half 176 to be further increased as the current passes through each of the "upper" MAGFETs 172, since each of the MAGFETs 172 will experience the same current-crowding effects as MAGFET 160. Conversely, the decreased current in split-drain half 186 of MAGFET 162 will be further decreased as this current passes through each of the "lower" MAGFETs 174, which will also experience the current-crowding effects resulting from the external magnetic field. Thus, the current at terminal IH in Fig. 12 will be increased by an amount reflecting the increase in current through split-drain halves in each of the "upper" MAGFETs 172; current at terminal IL in Fig. 12 will be decreased by an amount reflecting the decrease in current through split-drain halves in each of the "lower" MAGFETs 174. The resulting differential between the currents at IH and IL will thus reflect the additive effects of current crowding through each of the "upper" MAGFETs 172 and "lower" MAGFETs 174.

It is contemplated by the inventors that the current differential between IH and IL resulting from application of an external magnetic field to the circuit of Fig. 12 can be utilized to control the duty cycle of a postive-feedback oscillator, well known in the art of circuit design. In this way, the application of a magnetic field can be indicated by a change in the duty cycle of the oscillator. It is anticipated that the change in the average voltage of the duty cycle modulated signal would be obtained by low-pass filtering of the signal. No trim would be required in this embodiment.

In each of the embodiments of the present invention disclosed herein, it is contemplated that the MAGFET device utilized may be implemented as a separate component of an implantable medical device, such as an implantable pacemaker, cardioverter, defibrillator, neural stimulator, or the like, in order that the MAGFET device may be substituted for the reed switch in existing designs. Alternatively, the MAGFET device may be implemented in new designs as an integral part of the CMOS integrated circuit typically included in such devices. In either case, certain constraints exist with respect to the packaging of the MAGFET circuits and placement of the MAGFET circuit within the implantable device's enclosure.

Although CMOS integrated circuits are commonly packaged in Kovar-covered ceramic leadless chip carriers, such an arrangement may not be suitable for circuits which incorporate a MAGFET circuit in accordance with the present invention, as such packaging may tend to interfere with or deflect an externally applied magnetic field. In addition, circuits containing MAGFET circuits in accordance with the present invention must be positioned within an implanted device away from other ferromagnetic materials which may also be contained therein.

From the foregoing description of specific embodiments of the present invention, it should be apparent that a solid-state magnetic sensor has been disclosed which due to its operability at low bias currents and supply voltages is particularly well-suited for use in an implantable medical device. While specific embodiments of the present invention have been disclosed in detail, it is to be understood that various alterations, modifications, or substitutions therein may be made without departing from the spirit and scope of the present invention, as defined in the claims, which follow.

## Claims

1. An implantable medical device, responsive to application of an external magnetic field to alter an aspect of its operation, comprising an integrated circuit implemented on a silicon substrate, said integrated circuit comprising:

   at least one first split-drain FET having a source, a gate defining a first planar channel region, and first and second split-drain parts;

   a second split-drain FET having a source, a gate defining a second planar channel region, and third and fourth split-drain parts; and

   a differential amplifier, said differential amplifier producing an output voltage;

   wherein said first split-drain part is coupled to said fourth split-drain part, and said second split-drain part is coupled to said third split-drain part;

   and wherein said source of said first split-drain FET is coupled to a power supply, and said source of said second split-drain FET is coupled to a current source, such that a bias current is conducted through said first, second, third, and fourth split-drain parts;

   and wherein a first portion of said bias current is conducted through said first split-drain part and said fourth split-drain part, and a second portion of said bias current is conducted through said second split-drain part and said third split-drain part;

   such that a magnetic field perpendicular to said first and second planar channel regions increases said first portion of said bias current and decreases said second portion of said bias current;

   said differential amplifier voltage changing in response to said increase in said first portion of said bias current and said decrease in said second portion of said bias current.

2. An implantable medical device as claimed in claim 1, wherein said first and second split-drain FETs have two split-drain halves or three split-drain thirds.

3. An implantable medical device as claimed in claim 1 or 2 wherein said differential amplifier comprises a plurality of FET devices.

4. An implantable medical device as claimed in any preceding claim, wherein said first split-drain FET is a P-channel split-drain FET, and wherein said second split-drain FET is an N-channel FET.

5. An implantable medical device as claimed in any preceding claim, wherein said bias current is in a range from ten to one-hundred nano-amps.

6. An implantable medical device as claimed in any preceding claim, wherein said first and second planar channel regions have width-to-length ratios of 1:1.

7. An implantable medical device as claimed in any preceding claim, further comprising:

   a multiplexed active load circuit receiving a plurality of digital input signals and having a plurality of output lines, said multiplexed active load circuit comprising a plurality of said first split-drain FETs, said digital input signals for selecting one of said plurality of split-drain FETs to be coupled to said plurality of output lines, each of said plurality of split-drain FETs having a source, a gate defining a planar channel region, and first and second split-drain halves, said planar channel region of each of said plurality of split-drain FETs having different dimensions from others of said plurality of split-drain FETs;

and wherein said sources of said plurality of split-drain FETs comprising said multiplexed active load are coupled to the power supply, said second split-drain FET comprising a differential pair and said source of said second split-drain FET being coupled to the current source, such that a bias current is conducted through said first and second split-drain halves of said selected split-drain FET and said third and fourth split-drain halves;

and wherein a first portion of said bias current is conducted through said first split-drain half of said selected split-drain FET and said fourth split-drain half, and a second portion of said bias current is conducted through said second split-drain half of said selected split-drain FET and said third split-drain half;

such that a magnetic field perpendicular to said planar channel region of said selected split-drain FET and said second planar channel region increases said first portion of said bias current and decreases said second portion of bias current;

said differential amplifier output voltage changing in response to said increase in said first portion of bias current and said decrease in said second portion of said bias current, the extent of said change in differential amplifier output voltage varying depending upon which of said plurality of split-drain FETs is selected.

8. An implantable medical device, responsive to application of an external magnetic field to alter an aspect of its operation, comprising an integrated circuit implemented on a silicon substrate, said integrated circuit comprising:

a split-drain FET having a source, a gate defining a first planar channel region, and first and second split-drain halves, said split-drain FET being coupled to a bias circuit including a power supply such that a bias current flows through said first and second split-drain halves; and

a plurality of FET devices comprising a differential amplifier coupled between said first and second split-drain halves, said differential amplifier producing an output voltage;

wherein an external magnetic field applied to said split-drain FET increases said bias current in said first split-drain half and decreases the bias current in said second split-drain half;

and wherein said differential amplifier output voltage varies in proportion to the increase in bias current in said first split-drain half and said decrease in bias current in said second split-drain half;

such that said application of said external magnetic field is manifested by said variation in said differential amplifier output voltage.

9. An implantable medical device as claimed in claim 8 wherein said bias current is in a range from ten to one-hundred nano-amps.

10. An implantable medical device as claimed in claim 8 or 9 wherein said planar channel region has a width-to-length ratio of 1:1.

11. An implantable medical device, responsive to application of an external magnetic field to alter an aspect of its operation, comprising an integrated circuit implemented on a silicon substrate, said integrated circuit comprising:

a tri-drain FET having a source, a gate defining a first planar channel region, and a split drain having first, second, and third tri-drain thirds, said tri-drain FET being coupled to a bias circuit including a power supply such that a bias current is established in said tri-drain FET, a portion of said bias current being conducted through each of said first, second, and third tri-drain thirds;

a plurality of FET devices comprising a differential amplifier coupled between said first and third split-drain thirds, said differential amplifier producing an output voltage;

wherein an external magnetic field applied to said split-drain FET increases said bias current in said first tri-drain third and decreases the bias current in said third tri-drain third;

and wherein said differential amplifier output voltage varies in proportion to the increase in bias current in said first tri-drain third and said decrease in bias current in said third tri-drain third;

such that the presence of said external magnetic field is manifested by said variation in said differential amplifier output voltage.

12. An implantable medical device in accordance with claim 11, further comprising:

an active load comprising a second tri-drain FET having fourth, fifth, and sixth tri-drain thirds,

said fourth tri-drain third being coupled to said first tri-drain third, said fifth tri-drain third being coupled to said second tri-drain third, and said sixth tri-drain third being coupled to said third tri-drain third;

said second tri-drain FET being coupled to said bias circuit such that said portion of said bias current conducted through said first tri-drain third is also conducted through said fourth tri-drain third, said portion of said bias current conducted through said second tri-drain third is also conducted through said fifth tri-drain third, and said portion of said bias current conducted through said third tri-drain third is also conducted through said sixth tri-drain third;

such that said external magnetic field increases said portion of said bias current conducted through said first and fourth tri-drain thirds, and decreases said portion of said current conducted through said third and sixth tri-drain thirds;

and such that said differential amplifier output voltage varies in proportion to said increase in bias current in said first and fourth tri-drain thirds and said decrease in bias current in said third and sixth tri-drain thirds.

13. An implantable medical device responsive to an externally applied magnetic field to change an aspect of its operation, comprising an integrated circuit implemented on a silicon substrate, said integrated circuit comprising:

a first plurality of cascoded split-drain FETs coupled to a regulated current drain, and a second plurality of cascoded split-drain FETs coupled to said first plurality of split-drain FETs and said regulated current drain, said first plurality of cascoded split-drain FETs producing a first output current and said second plurality of cascoded split-drain FETs producing a second output current;

a positive-feedback oscillator producing an oscillating output having a duty cycle which varies in proportion to said first and second output currents;

wherein an external magnetic field applied to said integrated circuit increases said first output current and decreases said second output current;

such that said application of said external magnetic field alters said duty cycle of said oscillating signal.

14. An implantable medical device as claimed in claim 13, further comprising a low-pass filter for receiving said oscillating signal, said low-pass filter producing an output voltage proportional to the average voltage of said oscillating signal, such that a change in said duty cycle of said oscillating signal produces a change in said output voltage.

15. An implantable medical device including a circuit responsive to application of an external magnetic field comprising a first P-channel FET having first and second split-drain parts and a second N-channel FET having third and fourth split-drain parts, said first and fourth drain parts being connected and said FETs being arranged and biassed such that an applied magnetic field perpendicular in one direction to the integrated circuit causes an increase in the current in said first drain and a decrease in the current in said fourth drain, accompanied by a corresponding respective decrease and increase in the current in said second and third drain parts, said circuit further comprising a means for monitoring the current change in some or all of the drain parts to determine the application of a magnetic field.

16. An implantable medical device as claimed in claim 15 wherein said second and third drain parts are connected and said monitoring means monitors the voltage difference between the connected drain parts.

17. An implantable medical device as claimed in claim 16 wherein said monitoring means comprises an oscillator means connected to said second and third drain parts which provides a duty cycle which varies in response to the current in said second and third drain parts.

Fig. 1a

Fig. 1b

Fig. 2a

Fig. 2b

Fig. 3

Fig. 4

Fig. 5

Fig. 6

Fig. 7

EP 0 530 006 A1

EP 0 530 006 A1

Fig. 8

Fig. 9

140

VDD ... VDD ... VDD

LD1          LD2          LD3

Fig. 10

DIF1      DIF2      DIF3      142

DIFO

Fig. 11

Fig. 12

European Patent Office

**EUROPEAN SEARCH REPORT**

Application Number

EP    92 30 7763

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 096 190 (LGZ LANDIS & GYR ZUG AG)  * the whole document * | 1,8,11,13 | A61N1/372 H01L27/22 |
| A | | 2-7,9,10,15 | |
| Y,D | US-A-4 301 804 (THOMPSON ET AL.)  * the whole document * | 1,8,11,13 | |
| A | IEEE JOURNAL OF SOLID-STATE CIRCUITS vol. 18, no. 4, August 1988, NEW YORK US pages 426 - 428 R.S. POPOVIC ET AL 'A CMOS Magnetic Field Sensor'  * the whole document * | 1 | |
| A | US-A-3 829 883 (R.T. BATE)  * the whole document * | 2 | |
| A,D | IEEE JOURNAL OF SOLID-STATE CIRCUITS vol. 22, no. 2, April 1987, NEW YORK US pages 230 - 232 A. NATHAN ET AL 'Design of a CMOS oscillator with Magnetic-Field frequency modulation'  * the whole document * | 13 | TECHNICAL FIELDS SEARCHED (Int. Cl.5)  A61N H01L A61B |

The present search report has been drawn up for all claims

| Place of search | Date of completion of the search | Examiner |
|---|---|---|
| THE HAGUE | 04 NOVEMBER 1992 | FERRIGNO A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document